# EUROPÄISCHE PATENTSCHRIFT

(11) **EP 3 451 286 B1**
(45) Veröffentlichungstag und Bekanntmachung des Hinweises auf die Patenterteilung: **28.08.2019**
(21) Anmeldenummer: 17188583.3
(22) Anmeldetag: 30.08.2017
(51) Int. Cl.: G06T 7/10, G06T 7/149, G06T 7/187

(54) **VERFAHREN ZUM SEGMENTIEREN EINER ORGANSTRUKTUR EINES UNTERSUCHUNGSOBJEKTS IN MEDIZINISCHEN BILDDATEN**
METHOD FOR SEGMENTING AN ORGAN STRUCTURE OF AN OBJECT UNDER INVESTIGATION IN MEDICAL IMAGE DATA
PROCÉDÉ DE SEGMENTATION D'UNE STRUCTURE ORGANIQUE D'UN OBJET D'EXAMEN DANS DES DONNÉES D'IMAGES MÉDICALES

(43) Veröffentlichungstag der Anmeldung: 06.03.2019
(73) Patentinhaber: Siemens Healthcare GmbH, 91052 Erlangen (DE)
(72) Erfinder: Kartmann, René, 90491 Nürnberg (DE)

(56) Entgegenhaltungen:
- GB-A- 2 539 246
- US-A1- 2016 292 859
- ALJABAR P ET AL: "Multi-atlas based segmentation of brain images: Atlas selection and its effect on accuracy", NEUROIMAGE, ELSEVIER, AMSTERDAM, NL, Bd. 46, Nr. 3, 1. Juli 2009 (2009-07-01), Seiten 726-738, XP027453029, ISSN: 1053-8119, DOI: 10.1016/J.NEUROIMAGE.2009.02.018 [gefunden am 2009-02-23]
- GAO QINQUAN ET AL: "Modeling of the bony pelvis from MRI using a multi-atlas AE-SDM for registration and tracking in image-guided robotic prostatectomy", COMPUTERIZED MEDICAL IMAGING AND GRAPHICS, PERGAMON PRESS, NEW YORK, NY, US, Bd. 37, Nr. 2, 19. Februar 2013 (2013-02-19), Seiten 183-194, XP028590689, ISSN: 0895-6111, DOI: 10.1016/J.COMPMEDIMAG.2013.01.001
- PHAM D L ET AL: "CURRENT METHODS IN MEDICAL IMAGE SEGMENTATION", ANNUAL REVIEW OF BIOMEDICAL ENGINEE, ANNUAL REVIEW INCO., PALO ALTO, CA, US, Bd. 2, 1. August 2000 (2000-08-01), Seiten 315-337, XP009062827, DOI: 10.1146/ANNUREV.BIOENG.2.1.315

## Beschreibung

Die Erfindung betrifft ein Verfahren zum Segmentieren einer Organstruktur eines Untersuchungsobjekts in medizinischen Bilddaten, eine Recheneinheit, ein medizinisches Bildgebungsgerät und ein Computerprogrammprodukt

Üblicherweise werden medizinische Bilddaten mittels medizinischer Bildgebungsgeräte aufgenommen und können anatomische Strukturen und/oder funktionelle Vorgänge eines Körpers eines Patienten darstellen. Die Segmentierung von medizinischen Bilddaten gehört zu den am häufigsten eingesetzten Methoden zur Nachverarbeitung von medizinischen Bilddaten. Eine in den medizinischen Bilddaten segmentierte Organstruktur kann die Grundlage für eine computergestützte Auswertung der medizinischen Bilddaten darstellen. So ist beispielsweise eine automatische Erkennung von Pathologien der Organstruktur anhand von in der Segmentierung erkannten morphologischen Parametern denkbar. Weiterhin kann die Segmentierung der Organstruktur die Grundlage für eine Visualisierung der Organstruktur darstellen. Häufig ist auch die automatische Segmentierung von einem Zielorgan und/oder Risikoorgan auch im Workflow einer Planung einer Strahlentherapie sinnvoll einsetzbar.

Es sind verschiedene Algorithmen zur automatischen computergestützten Segmentierung von Organstrukturen in medizinischen Bilddaten bekannt. Diese Algorithmen sehen als Eingangsparameter selbstverständlich die medizinischen Bilddaten selbst bzw. aus den medizinischen Bilddaten abgeleitete Informationen, beispielsweise Texturparameter, vor. Weiterhin können patientenspezifische Eigenschaften, wie beispielsweise eine Größe, ein Alter oder ein Geschlecht des Patienten als Eingangsdaten in den Segmentierungsalgorithmus eingehen. Beispielsweise kann bei einer atlas-basierten Segmentierung ein geeigneter Atlas zur Segmentierung einer Organstruktur anhand des Geschlechts des Patienten ausgewählt werden. Verschiedene Methoden zur Segmentierung von medizinischen Bilddaten sind beispielsweise aus der US 20170011526 A1, der US 8170330 B2, der US 8837771 B2 oder der US 9367924 B2 bekannt.

Derzeit entwickeln sich molekulardiagnostische Methoden sehr rasch weiter, so dass eine Analyse des menschlichen Genoms immer weniger Zeit in Anspruch nimmt und kostengünstiger wird. Derart wird routinemäßige Verfügbarkeit von genetischen Daten zu einzelnen Patienten in der Zukunft deutlich höher sein. Derart können von einem Patienten neben medizinischen Bilddaten auch genetische Daten vorliegen, damit ein komplexes Krankheitsbild mittels verschiedener diagnostischer Parameter untersucht werden kann.

Aus der US 2016/0292859 A1 ist es bekannt, dass morphometrische Unterschiede mit natürlich vorkommenden genetischen Unterschieden assoziiert sein können und dass ein für eine Segmentierung verwendeter Atlas für eine bestimmte Populationsgruppe modelliert werden kann.

Aus der Schrift von Aljabar et al. "Multi-atlas based segmentation of brain images: Atlas selection and its effect on accuracy", Neuroimage 46 (2009) 726-738 ist es bekannt, dass für eine multi-atlas Segmentierung ein Atlas basierend auf einer Übereinstimmung des Atlas-Subjekts mit bestimmten Suchkriterien, beispielsweise in Bezug auf eine genetische Variable, übereinstimmt.

Aus der Schrift von Gao et al. "Modeling of the bony pelvis from MRI using a multi-atlas AE-SDM for registration and tracking in image-guided robotic prostatectomy", Computerized Medical Imaging and Graphics 37 (2013) 183-194 ist eine auf dem Geschlecht des Untersuchungsobjekts basierende Auswahl eines Atlas bekannt.

Der Erfindung liegt die Aufgabe zu Grunde, eine effektive und spezifisch auf das Untersuchungsobjekt abgestimmte Segmentierung einer Organstruktur des Untersuchungsobjekts zu ermöglichen. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zum Segmentieren einer Organstruktur eines Untersuchungsobjekts in medizinischen Bilddaten umfasst folgende Verfahrensschritte:
- Erfassen von genetischen Daten des Untersuchungsobjekts, welcher eine morphologische Variation der Organstruktur charakterisieren,
- Erfassen von medizinischen Bilddaten vom Untersuchungsobjekt,
- Segmentieren der Organstruktur in den medizinischen Bilddaten mittels eines Segmentierungsalgorithmus, wobei die genetischen Daten zusätzlich zu den medizinischen Bilddaten als Eingangsparameter in den Segmentierungsalgorithmus eingehen und wobei der Segmentierungsalgorithmus bei der Segmentierung der Organstruktur die morphologische Variation der Organstruktur berücksichtigt,
- Bereitstellen der segmentierten Organstruktur.

Die Organstruktur kann dabei ein gesamtes Körperorgan des Untersuchungsobjekts oder ein Teil eines Körperorgans des Untersuchungsobjekts sein. Das Untersuchungsobjekt kann ein Patient, ein gesunder Proband oder ein Tier sein. Das Erfassen der medizinischen Bilddaten kann eine Akquisition der medizinischen Bilddaten mittels eines medizinischen Bildgebungsgeräts oder ein Laden von bereits akquirierten medizinischen Bilddaten aus einer Bilddatenbank umfassen.

Das Erfassen der genetischen Daten kann eine Analyse des Genoms des Untersuchungsobjekts, beispielsweise mittels einer Gen-Sequenzierungsmethode, oder ein Laden von bereits analysierten genetischen Daten des Untersuchungsobjekts aus einer Datenbank umfassen. Die genetischen Daten betreffen insbesondere die für das Untersuchungsobjekt spezifischen Erbmerkmale, beispielsweise ein Teil einer DNA-Sequenz des Untersuchungsobjekts bzw. ein Teil des genetischen Fingerabdrucks des Untersuchungsobjekts. Die tatsächlich vom Untersuchungsobjekt erfassten genetischen Daten können so von der klinischen Fragestellung, insbesondere der zu segmentierenden Organstruktur, abhängen. Das Erfassen der genetischen Daten kann umfassen, dass ein für das Untersuchungsobjekt spezifischer genetischer Marker festgestellt wird, welcher geeignet als Eingangsparameter für den Segmentierungsalgorithmus verwendet werden kann. Insbesondere werden solche genetischen Daten des Untersuchungsobjekts erfasst, welche sich spezifisch auf eine Morphologie, beispielsweise eine Größe und/oder eine Form, der zu segmentierenden Organstruktur beziehen. Derart können die genetischen Daten beispielsweise charakterisieren, ob oder in welchem Maße die Organstruktur von der morphologischen Variation betroffen ist. Die morphologische Variation der Organstruktur ist dabei insbesondere eine Veränderung einer Morphologie, also beispielsweise einer Größe und/oder Form der Organstruktur, gegenüber einer Norm-Morphologie der Organstruktur. Das Erfassen der genetischen Daten kann dabei eine Prüfung, ob eine bestimmte genetische Variante, welche zu der morphologischen Variation der Organstruktur führt, in den genetischen Daten des Untersuchungsobjekts vorliegt, umfassen. Die genetische Variante, auch Genvariante genannt, ist dabei insbesondere eine Veränderung einer DNA-Sequenz des Untersuchungsobjekts gegenüber einer Norm-DNA-Sequenz, beispielsweise in einem Bereich zwischen Genen (intergenetische Variante, intergenetic variant).

Die Segmentierung der Organstruktur umfasst insbesondere eine automatische oder semiautomatische Erkennung der Organstruktur in den medizinischen Bilddaten. Die Segmentierung der Organstruktur kann eine Bestimmung umfassen, welcher Teil der medizinischen Bilddaten der Organstruktur zuzuordnen ist. Derart umfasst die Segmentierung der Organstruktur insbesondere eine Festlegung für jedes Voxel der medizinischen Bilddaten, ob das Voxel zur Organstruktur gehört oder nicht. Die Segmentierung der Organstruktur wird dabei insbesondere durch den Segmentierungsalgorithmus durchgeführt. Der Segmentierungsalgorithmus kann dabei eine bekannte Segmentierungsmethode einsetzen, beispielsweise eine atlas-basierte Segmentierung, ein Random-Walker Verfahren, ein Region-Growing Verfahren oder einen Einsatz eines künstlichen neuronalen Netzes. Es sind weitere Segmentierungsmethoden möglich, beispielsweise eine aktive Konturen Segmentierungsmethode (z.B. active snakes), eine Level-Set Segmentierungsmethode, oder eine statistische Segmentierungsmethode (z.B. active shape models). Selbstverständlich sind weitere, dem Fachmann als sinnvoll erscheinende Segmentierungsmethoden denkbar. Bei einer semi-automatischen Segmentierung kann der Benutzer die Segmentierung initialisieren, beispielsweise über das Setzen eines Saatpunkts und/oder über das Setzen zumindest einer Landmarke. Der Benutzer kann die erfolgte Segmentierung auch kontrollieren und/oder verändern.

Der Segmentierungsalgorithmus weist als Eingangsparameter sowohl die medizinischen Bilddaten, als auch die genetischen Daten auf. Dass der Segmentierungsalgorithmus die genetischen Daten als Eingangsparameter aufweist kann auch bedeuten, dass der Segmentierungsalgorithmus eine aus den genetischen Daten des Untersuchungsobjekts abgeleitete Information, insbesondere in Bezug auf die morphologische Variation der Organstruktur, als Eingangsparameter aufweist. Die Berücksichtigung der morphologischen Variation der Organstruktur erfolgt insbesondere basierend auf den genetischen Daten, welche beispielsweise angeben, ob oder in welchem Maße die Organstruktur von der morphologischen Variation betroffen ist.

Die genetischen Daten und die damit verknüpfte morphologische Variation der Organstruktur können derart besonders vorteilhafte Zusatzinformationen für die Segmentierung der Organstruktur darstellen. Wie in folgenden Ausführungsformen noch genauer beschrieben, kann gemäß den genetischen Daten der Segmentierungsalgorithmus besonders geeignet ausgewählt oder angepasst werden. Unter Berücksichtigung der genetischen Daten kann die Segmentierung der Organstruktur somit individuell auf das Untersuchungsobjekt abgestimmt erfolgen. Derart ist beispielsweise eine präzisere und/oder effektivere Segmentierung der Organstruktur denkbar. Es werden vorteilhafterweise nicht nur die Bildinhalte der medizinischen Bilddaten bei der Segmentierung der Organstruktur berücksichtigt, sondern auch eine durch die genetischen Daten charakterisierte Morphologie der Organstruktur. Beispielsweise ist es derart denkbar, dass ein Rechenaufwand der Segmentierung verringert werden kann, wenn auf Grundlage der durch die genetischen Daten charakterisierten morphologischen Variation bereits geeignete Randbedingungen für die Segmentierung gesetzt werden können.

Das Bereitstellen der segmentierten Organstruktur umfasst insbesondere eine Ausgabe der segmentierten Organstruktur auf einer Ausgabeeinheit, beispielsweise einer Anzeigeeinheit, und/oder ein Abspeichern der segmentierten Organstruktur in einer Datenbank. Die segmentierte Organstruktur wird dabei insbesondere in Bezug auf die medizinischen Bilddaten bereitgestellt, beispielsweise in den medizinischen Bilddaten geeignete unterscheidbar, z.B. farbkodiert, dargestellt. Die segmentierte Organstruktur kann alternativ oder zusätzlich an eine Weiterverarbeitungseinheit übergeben werden, welche basierend auf der segmentierten Organstruktur eine Weiterverarbeitung der medizinischen Bilddaten durchführen kann. Für den Anwendungsfall der Planung einer Bestrahlung des Untersuchungsobjekts kann die segmentierte Organstruktur als Zielorgan oder Risikoorgan für die Bestrahlungsplanung gesetzt werden.

Die Weiterverarbeitung der medizinischen Bilddaten bzw. der segmentierten Organstruktur kann wiederum unter Einsatz der genetischen Daten erfolgen. Beispielsweise kann ein auf die morphologische Variation des Untersuchungsobjekts abgestimmter Normwertebereich für eine Morphologie der Organstruktur in Abhängigkeit der genetischen Daten festgelegt werden. Dieser auf die genetischen Daten abgestimmte Normwertebereich kann geeignet bei einer automatischen Diagnose einer abnormalen Morphologie der Organstruktur, welche auf eine Erkrankung hinweisen kann, verwendet werden.

Eine Ausführungsform sieht vor, dass die morphologische Variation zumindest eines der folgenden morphologischen Merkmale der Organstruktur betrifft:
- eine Größe der Organstruktur,
- eine Form der Organstruktur,
- ein Volumen der Organstruktur,
- eine Lokalisation der Organstruktur im Körper des Untersuchungsobjekts.

Derart kann der Segmentierungsalgorithmus, wenn die morphologische Variation aus den genetischen Daten bekannt ist, besonders geeignet das für das Untersuchungsobjekt spezifische zumindest eine morphologische Merkmal berücksichtigen. Die durch die genetischen Daten charakterisierte morphologische Variation kann dabei eine Information umfassen, in welchem Maße das zumindest eine morphologische Merkmal von der morphologischen Variation betroffen ist. Dieses Ausmaß der Veränderung des zumindest einen morphologischen Merkmals kann dabei absolut oder relativ zu einer anderen Organstruktur bzw. zu einem Normwertebereich für das morphologische Merkmal angegeben werden. Ein aus den genetischen Daten bekanntes Vorwissen über zumindest eines der genannten morphologischen Merkmale kann besonders geeignet als zusätzliche Informationen bei der Segmentierung der Organstruktur unterstützen.

Erfindungsgemäß umfasst das Erfassen der genetischen Daten eine Prüfung, ob eine genetische Variante, welche zu der morphologischen Variation der Organstruktur führt, in den genetischen Daten des Untersuchungsobjekts vorliegt, wobei ein Ergebnis der Prüfung als Eingangsparameter in den Segmentierungsalgorithmus eingeht und der Segmentierungsalgorithmus bei der Segmentierung der Organstruktur das Ergebnis der Prüfung berücksichtigt.

Typischerweise führt die genetische Variante, beispielsweise eine intergenetische Variante, zu der morphologischen Variation. Das heißt insbesondere, dass nur wenn in den genetischen Daten die genetische Variante vorliegt, die Organstruktur von der morphologischen Variation betroffen ist. Derart kann mittels der Prüfung, ob die genetische Variante in den genetischen Daten vorliegt, besonders vorteilhaft die morphologische Variation der Organstruktur bestimmt werden. Das Ergebnis der Prüfung kann dabei insbesondere eine binäre Information sein, ob die genetische Variante in den genetischen Daten des Untersuchungsobjekts vorliegt oder nicht. Die binäre Information kann derart geeignet als Zusatzinformation in die Segmentierung der Organstruktur eingehen. Beispielsweise kann auf Grundlage der binären Information der Segmentierungsalgorithmus ausgewählt und/oder ein Segmentierungsparameter des Segmentierungsalgorithmus gesetzt oder angepasst werden.

Eine Ausführungsform sieht vor, dass in einem weiteren Verfahrensschritt zunächst festgelegt wird, welcher Organstruktur-Typ in den medizinischen Bilddaten segmentiert werden soll, und wobei die Prüfung gemäß dem festgelegten Organstruktur-Typ erfolgt.

Derart wird insbesondere zuerst festgelegt bzw. festgestellt, welcher Organstruktur-Typ, d.h. insbesondere welche Art von Organstruktur, segmentiert werden soll und basierend auf dieser Feststellung geprüft, ob in den genetischen Daten des Patienten eine genetische Variante vorliegt, welche spezifisch den zu einer morphologischen Veränderung des zu segmentierenden Organstruktur-Typs führt. Derart können die genetischen Daten des Untersuchungsobjekts besonders gezielt nach für den Organstruktur-Typ entscheidenden genetischen Varianten durchsucht werden.

Alternativ ist auch das Vorgehen denkbar, dass vor der Segmentierung unabhängig von dem zu segmentierenden Organstruktur-Typ geprüft wird, ob eine auffällige genetische Variante, welche Organstrukturen in ihrer Morphologie beeinflussen kann, in den genetischen Daten des Patienten vorliegt, so dass dementsprechend eine geeignete Anpassung der durchzuführenden Segmentierung erfolgen kann.

Eine Ausführungsform sieht vor, dass das Erfassen der genetischen Daten ein Erfassen einer Information umfasst, in welchem Maße die Organstruktur von der morphologischen Variation in ihrer Morphologie verändert ist, wobei die Information als Eingangsparameter in den Segmentierungsalgorithmus eingeht und der Segmentierungsalgorithmus bei der Segmentierung der Organstruktur die Information berücksichtigt.

Derart kann der Segmentierungsalgorithmus besonders geeignet gemäß dem Maß der Veränderung der Morphologie der Organstruktur durch die morphologische Variation angepasst werden. Das Maß der Veränderung kann dabei in Prozent zu Normwerten der Morphologie der Organstruktur angegeben werden.

Es wäre auch denkbar, dass der Segmentierungsalgorithmus eine atlas-basierte Segmentierung unter Verwendung eines Atlas einsetzt, wobei der für die Segmentierung verwendete Atlas gemäß dem Vorliegen der durch die genetischen Daten charakterisierten morphologischen Variation aus einer Menge an Atlanten ausgewählt wird.

Die Menge von Atlanten kann einen ersten Atlas und einen zweiten Atlas umfassen, wobei der erste Atlas auf Atlas-Bilddaten eines ersten Atlas-Kollektivs basiert, welches eine mit der morphologischen Variation verknüpfte genetische Variante aufweist, und wobei der zweite Atlas auf Atlas-Bilddaten eines zweiten Atlas-Kollektivs basiert, welches die mit der morphologischen Variation verknüpfte genetische Variante nicht aufweist. Gemäß dem Vorliegen der genetischen Variante bzw. der morphologischen Variation der Organstruktur kann dann der geeignete Atlas für die Segmentierung der Organstruktur aus der Menge der Atlanten ausgewählt werden. Die Verwendung des geeignet auf die morphologische Variation der Organstruktur abgestimmten Atlas kann die atlas-basierte Segmentierung genauer und/oder performanter machen.

Es wäre auch denkbar, dass der Segmentierungsalgorithmus eine atlas-basierte Segmentierung unter Verwendung eines Atlas einsetzt, wobei der Atlas zumindest eine Atlas-Organstruktur aufweist und die Atlas-Organstruktur gemäß dem Vorliegen der durch die genetischen Daten charakterisierten morphologischen Variation deformiert wird.

Derart kann zunächst ein Standard-Atlas oder ein gemäß der vorherigen Ausführungsform geeignet ausgewählter Atlas hinzugezogen werden. Dieser Atlas kann dann vor seiner Verwendung für die Segmentierung, beispielsweise vor der Registrierung auf die medizinischen Bilddaten, geeignet anhand der genetischen Daten angepasst werden. Beispielsweise kann zumindest eine Atlas-Organstruktur im Atlas gemäß der durch die genetischen Daten charakterisierten morphologischen Variation deformiert werden. Mittels des derart angepassten Atlas ist eine genaue und/oder performante atlas-basierte Segmentierung der Organstruktur möglich.

Es wäre auch denkbar, dass der Segmentierungsalgorithmus ein Region-Growing Verfahren oder ein Random-Walker Verfahren unter Verwendung einer Randbedingung für die Segmentierung der Organstruktur einsetzt, wobei die Randbedingung gemäß der durch die genetischen Daten charakterisierten morphologischen Variation festgelegt wird.

Wenn die morphologische Variation eine Veränderung zumindest eines morphologischen Merkmals angibt, kann die Veränderung des zumindest einen morphologischen Merkmals eine geeignete Randbedingung für das Region-Growing Verfahren oder das Random-Walker Verfahren darstellen. Die genannten Segmentierungsverfahren können durch die Verwendung der Randbedingung, welche perfomanter ablaufen und genauere Ergebnisse bereitstellen.

Erfindungsgemäß setzt der Segmentierungsalgorithmus ein für die Segmentierung der Organstruktur trainiertes künstliches neuronales Netz ein, wobei das für die Segmentierung verwendete künstliche neuronale Netz gemäß dem Vorliegen der durch die genetischen Daten charakterisierten morphologischen Variation ausgewählt und/oder verändert wird.

Derart basiert die Segmentierung der Organstruktur insbesondere auf einem maschinellen Lernverfahren, auch Deep-Learning Verfahren genannt, welches auf dem künstlichen neuronalen Netz basiert. Ein künstliches neuronales Netz (KNN, englisch artificial neural network - ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen.

Das künstliche neuronale Netz wurde insbesondere im Vorfeld bereits geeignet für die Segmentierung der Organstruktur trainiert. Für das Training des künstlichen neuronalen Netzes wurden dabei insbesondere medizinische Trainingsbilddatensätze verwendet, in welchen die Organstruktur bereits segmentiert vorliegt. Die medizinischen Trainingsdatensätze sind dabei typischerweise von vom Untersuchungsobjekt verschiedenen Trainings-Untersuchungsobjekten akquiriert worden.

Mittels der Berücksichtigung der genetischen Daten kann nun die Segmentierung mittels des künstlichen neuronalen Netzes spezifisch auf das Untersuchungsobjekt abgestimmt werden. Beispielsweise kann ein geeignet trainiertes künstliches neuronales Netz anhand des Vorliegens einer Genvariante beim Untersuchungsobjekt ausgewählt werden, wie in der folgenden Ausführungsform noch genauer beschrieben. Auch ist es denkbar, dass das trainierte künstliche neuronale Netz geeignet anhand der genetischen Daten nachträglich angepasst wird, so dass das künstliche neuronale Netz die Segmentierung besonders vorteilhaft auf die durch die genetischen Daten charakterisierte morphologische Variation abgestimmt durchführen kann. Auch ist es denkbar, dass die genetischen Daten oder eine aus den genetischen Daten abgeleitete Information als zusätzlicher Trainingsparameter beim Training des künstlichen neuronalen Netzes berücksichtigt werden. Um die Komplexität zu reduzieren, ist es beispielsweise vorteilhaft aus den genetischen Daten einen binären Trainingsparameter, ob eine bestimmte Genvariante beim Untersuchungsobjekts vorliegt oder nicht, abzuleiten und diesen beim Training des künstlichen neuronalen Netzes einzusetzen.

Erfindungsgemäß stehen für die Segmentierung der Organstruktur ein erstes künstliches neuronales Netz und ein zweites künstliches neuronales Netz bereit, wobei das erste künstliche neuronale Netz mittels eines ersten Trainingskollektivs trainiert worden ist, welches die genetische Variante aufweist, und das zweite künstliche neuronale Netz mittels eines zweiten Trainingskollektivs trainiert worden ist, welches die genetische Variante nicht aufweist, wobei gemäß dem Ergebnis der Prüfung das erste künstliche neuronale Netz oder das zweite künstliche neuronale Netz für die Segmentierung der Organstruktur eingesetzt wird.

Ergibt die Prüfung, dass das Untersuchungsobjekt die genetische Variante aufweist, so wird insbesondere das erste künstliche neuronale Netz für die Segmentierung verwendet. Ergibt die Prüfung, dass das Untersuchungsobjekt die genetische Variante nicht aufweist, so wird insbesondere das zweite künstliche neuronale Netz für die Segmentierung verwendet. Das derart ausgewählte künstliche neuronale Netz kann die Segmentierung besonders präzise und/oder performant durchführen, da es besonders geeignet auf die durch die genetischen Daten charakterisierte morphologische Variation abgestimmt ausgewählt worden ist.

Eine Ausführungsform sieht vor, dass ein weiteres patientenspezifisches Merkmal des Untersuchungsobjekts erfasst wird, wobei das weitere patientenspezifische Merkmal zusätzlich zu den medizinischen Bilddaten und den genetischen Daten in den Segmentierungsalgorithmus eingeht und zumindest ein Merkmal aus der folgenden Liste umfasst:
- ein Alter des Untersuchungsobjekts,
- ein Geschlecht des Untersuchungsobjekts,
- eine Größe des Untersuchungsobjekts,
- ein Gewicht des Untersuchungsobjekts.

Das patientenspezifische Merkmal kann derart als besonders vorteilhafte weitere Zusatzinformation in die Segmentierung der Organstruktur eingehen. Derart kann die Segmentierung der Organstruktur noch weiter individuell auf das Untersuchungsobjekt abgestimmt durchgeführt werden.

Eine Ausführungsform sieht vor, dass die zu segmentierende Organstruktur eine der folgenden Organstrukturen ist:
- eine Gehirnstruktur des Untersuchungsobjekts,
- eine Prostata des Untersuchungsobjekts,
- eine Herzstruktur des Untersuchungsobjekts.

Für die genannten Organstrukturen kann das erfindungsgemäße Verfahren besonders geeignet angewandt werden. Die Gehirnstruktur kann beispielsweise eine oder mehrere Strukturen aus der folgenden Liste umfassen: Corpus Callosum, Hippocampus, Cortex, Thalamus, Hypothalamus, Hirnstamm, Kleinhirn, weiße Gehirnsubstanz, graue Gehirnsubstanz, Liquor cerebrospinalis, usw. Die Herzstruktur kann beispielsweise eine oder mehrere Strukturen aus der folgenden Liste umfassen: rechter Vorhof, linker Vorhof, rechter Ventrikel, linker Ventrikel, Aorta, Perikard, Myokard, Epikard, Herzspitze, usw. Selbstverständlich sind weitere Organstrukturen, welche mittels des erfindungsgemäßen Verfahrens segmentiert werden können, denkbar.

Die erfindungsgemäße Recheneinheit umfasst zumindest ein Rechenmodul, wobei die Recheneinheit zum Ausführen eines erfindungsgemäßen Verfahrens ausgebildet ist.

So ist insbesondere die Recheneinheit dazu ausgebildet, computerlesbare Instruktionen auszuführen, um das erfindungsgemäße Verfahren auszuführen. Insbesondere umfasst die Recheneinheit eine Speichereinheit, wobei auf der Speichereinheit computerlesbare Informationen gespeichert sind, wobei die Recheneinheit dazu ausgebildet ist, die computerlesbaren Informationen von der Speichereinheit zu laden und die computerlesbaren Informationen auszuführen, um ein erfindungsgemäßes Verfahren auszuführen.

Derart ist die erfindungsgemäße Recheneinheit dazu ausgebildet, ein Verfahren zum Segmentieren einer Organstruktur eines Untersuchungsobjekts in medizinischen Bilddaten auszuführen. Die Recheneinheit kann dafür eine erste Erfassungseinheit zum Erfassen von genetischen Daten des Untersuchungsobjekts, welche eine morphologische Variation der Organstruktur charakterisieren, umfassen. Die Recheneinheit kann eine zweite Erfassungseinheit zum Erfassen von medizinischen Bilddaten vom Untersuchungsobjekt umfassen. Die Recheneinheit kann eine Segmentierungseinheit zum Segmentieren der Organstruktur in den medizinischen Bilddaten mittels eines Segmentierungsalgorithmus, wobei die genetischen Daten zusätzlich zu den medizinischen Bilddaten als Eingangsparameter in den Segmentierungsalgorithmus eingehen und wobei der Segmentierungsalgorithmus bei der Segmentierung der Organstruktur die morphologische Variation der Organstruktur berücksichtigt, umfassen. Die Recheneinheit kann eine Bereitstellungseinheit zum Bereitstellen der segmentierten Organstruktur umfassen.

Die Komponenten der Recheneinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Das erfindungsgemäße medizinische Bildgebungsgerät umfasst die erfindungsgemäße Recheneinheit.

Die Recheneinheit kann dazu ausgebildet sein, Steuerungssignale an das medizinische Bildgebungsgerät zu senden und/oder Steuerungssignale zu empfangen und/oder zu verarbeiten, um ein erfindungsgemäßes Verfahren auszuführen. Die Recheneinheit kann in das medizinische Bildgebungsgerät integriert sein. Die Recheneinheit kann auch separat von dem medizinischen Bildgebungsgerät installiert sein. Die Recheneinheit kann mit dem medizinischen Bildgebungsgerät verbunden sein.

Das Erfassen der medizinischen Bilddaten kann eine Aufnahme der medizinischen Bilddaten mittels einer Aufnahmeeinheit des medizinischen Bildgebungsgeräts umfassen. Die medizinischen Bilddaten können dann an die Recheneinheit zur Weiterverarbeitung übergeben werden. Die Recheneinheit kann die medizinischen Bilddaten dann mittels der zweiten Erfassungseinheit erfassen.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile der erfindungsgemäßen Computerprogrammprodukts, des erfindungsgemäßen medizinischen Bildgebungsgeräts und der erfindungsgemäßen Recheneinheit entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:
- Fig. 1: ein medizinisches Bildgebungsgerät mit einer erfindungsgemäßen Recheneinheit,
- Fig. 2: eine erste Ausführungsform eines erfindungsgemäßen Verfahrens,
- Fig. 3: eine zweite Ausführungsform eines erfindungsgemäßen Verfahrens
- Fig. 4: eine erste mögliche Anwendung eines erfindungsgemäßen Verfahren,
- Fig. 5: eine zweite mögliche Anwendung eines erfindungsgemäßen Verfahren,
- Fig. 6: eine dritte mögliche Anwendung eines erfindungsgemäßen Verfahren und
- Fig. 7: eine vierte mögliche Anwendung eines erfindungsgemäßen Verfahren.

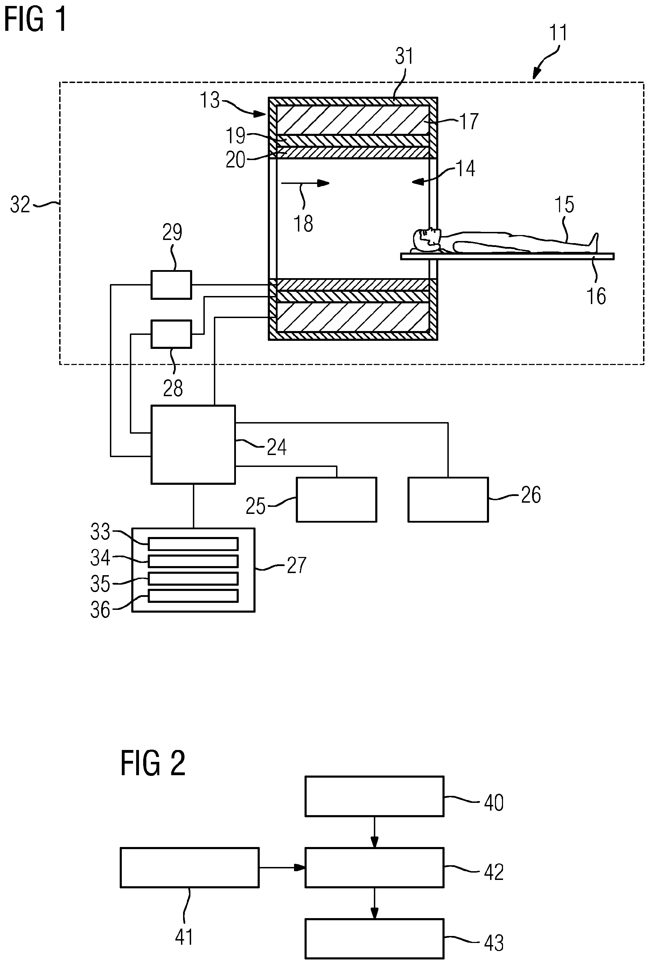

**Fig. 1** zeigt ein medizinisches Bildgebungsgerät 11 mit einer erfindungsgemäßen Recheneinheit 27.

Das medizinische Bildgebungsgerät 11 kann beispielsweise ein Magnetresonanzgerät, ein Einzelphotonenemissionstomographie-Gerät (SPECT-Gerät), ein Positronen-Emissions-Tomographie-Gerät (PET-Gerät), ein Computertomograph, ein Ultraschall-Gerät, ein Röntgengerät oder ein C-Bogen-Gerät sein. Es sind dabei auch kombinierte medizinische Bildgebungsgeräte 11 möglich, welche eine beliebige Kombination aus mehreren der genannten Bildgebungsmodalitäten umfassen.

Im gezeigten Fall ist das medizinische Bildgebungsgerät 11 exemplarisch als Magnetresonanzgerät 11 ausgebildet.

Das Magnetresonanzgerät 11 umfasst eine von einer Magneteinheit 13 gebildete Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Patienten 15, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Liegentisch auf, der bewegbar innerhalb des Magnetresonanzgeräts 11 angeordnet ist. Die Magneteinheit 13 ist mittels einer Gehäuseverkleidung 31 des Magnetresonanzgeräts nach außen abgeschirmt.

Die Magneteinheit 13 weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 10 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Magnetresonanz-Sequenzen in einen Untersuchungsraum, der im Wesentlichen von dem Patientenaufnahmebereich 14 gebildet ist, ein. Die Hochfrequenzantenneneinheit 20 ist weiterhin zum Empfang von Magnetresonanz-Signalen, insbesondere aus dem Patienten 15, ausgebildet.

Zu einer Steuerung des Hauptmagneten 17, der Gradientensteuereinheit 28 und der Hochfrequenzantennensteuereinheit 29 weist das Magnetresonanzgerät 11 eine Steuereinheit 24 auf. Die Steuereinheit 24 steuert zentral das Magnetresonanzgerät 11, wie beispielsweise das Durchführen einer vorbestimmten bildgebenden Gradientenechosequenz. Steuerinformationen wie beispielsweise Bildgebungsparameter, sowie rekonstruierte Magnetresonanz-Bilder können auf einer Bereitstellungseinheit 25, im vorliegenden Fall einer Anzeigeeinheit 25, des Magnetresonanzgeräts 11 für einen Benutzer bereitgestellt werden. Zudem weist das Magnetresonanzgerät 11 eine Eingabeeinheit 26 auf, mittels derer Informationen und/oder Parameter während eines Messvorgangs von einem Benutzer eingegeben werden können. Die Steuereinheit 24 kann die Gradientensteuereinheit 28 und/oder Hochfrequenzantennensteuereinheit 29 und/oder die Anzeigeeinheit 25 und/oder die Eingabeeinheit 26 umfassen.

Das Magnetresonanzgerät 11 umfasst weiterhin eine Aufnahmeeinheit 32. Die Aufnahmeeinheit 32 wird im vorliegenden Fall von der Magneteinheit 13 zusammen mit der Hochfrequenzantennensteuereinheit 29 und der Gradientensteuereinheit 28 gebildet.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

Das dargestellte Magnetresonanzgerät 11 umfasst eine Recheneinheit 27, welche eine erste Erfassungseinheit 33, eine zweite Erfassungseinheit 34, eine Segmentierungseinheit 35 und eine Bereitstellungseinheit 36 umfasst. Derart ist die Recheneinheit 27 zum Ausführen eines Verfahrens gemäß Fig. 2-3 ausgebildet.

Zum alleinigen Ausführen eines erfindungsgemäßen Verfahrens wird die Recheneinheit 27 vorteilhafterweise medizinische Bilddaten mittels der zweiten Erfassungseinheit 34 aus einer Datenbank laden. Wenn das erfindungsgemäße Verfahren kombiniert von dem Magnetresonanzgerät 11 und der Recheneinheit 27 ausgeführt wird, wird die zweite Erfassungseinheit 34 der Recheneinheit 27 insbesondere medizinische Bilddaten, welche mittels der Aufnahmeeinheit 32 des Magnetresonanzgeräts 11 aufgenommen worden sind, erfassen. Dafür ist die Recheneinheit 27, insbesondere die zweite Erfassungseinheit 34, vorteilhafterweise mit der Steuereinheit 24 des Magnetresonanzgeräts 11 hinsichtlich eines Datenaustauschs verbunden. Wenn das erfindungsgemäße Verfahren kombiniert von dem Magnetresonanzgerät 11 und der Recheneinheit 27 ausgeführt wird, kann die segmentierte Organstruktur, welche von der Recheneinheit 27 segmentiert wird, auf der Bereitstellungseinheit 25 des Magnetresonanzgeräts 11 bereitgestellt werden.

**Fig. 2** zeigt ein Ablaufdiagramm einer ersten Ausführungsform eines erfindungsgemäßen Verfahrens zum Segmentieren einer Organstruktur eines Untersuchungsobjekts 15 in medizinischen Bilddaten.

In einem ersten Verfahrensschritt 40 erfolgt ein Erfassen von genetischen Daten des Untersuchungsobjekts 15, welche eine morphologische Variation der Organstruktur charakterisieren. Die morphologische Variation kann dabei zumindest eines der folgenden morphologischen Merkmale der Organstruktur betreffen:
- eine Größe der Organstruktur,
- eine Form der Organstruktur,
- ein Volumen der Organstruktur,
- eine Lokalisation der Organstruktur im Körper des Untersuchungsobjekts.

In einem weiteren Verfahrensschritt 41 erfolgt ein Erfassen von medizinischen Bilddaten vom Untersuchungsobjekt 15.

In einem weiteren Verfahrensschritt 42 erfolgt ein Segmentieren der Organstruktur in den medizinischen Bilddaten mittels eines Segmentierungsalgorithmus, wobei die genetischen Daten zusätzlich zu den medizinischen Bilddaten als Eingangsparameter in den Segmentierungsalgorithmus eingehen und wobei der Segmentierungsalgorithmus bei der Segmentierung der Organstruktur die morphologische Variation der Organstruktur berücksichtigt.

In einem weiteren Verfahrensschritt 43 erfolgt ein Bereitstellen der segmentierten Organstruktur.

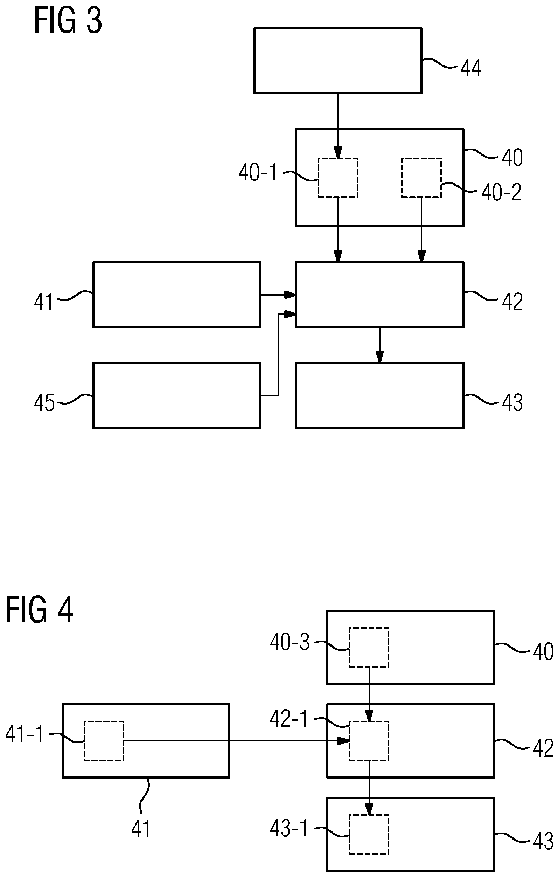

**Fig. 3** zeigt ein Ablaufdiagramm einer zweiten Ausführungsform eines erfindungsgemäßen Verfahrens Segmentieren einer Organstruktur eines Untersuchungsobjekts 15 in medizinischen Bilddaten.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

Die in Fig. 3 gezeigte Ausführungsform des erfindungsgemäßen Verfahrens umfasst im Wesentlichen die Verfahrensschritte 40, 41, 42, 43 der ersten Ausführungsform des erfindungsgemäßen Verfahrens gemäß Fig. 2. Zusätzlich umfasst die in Fig. 3 gezeigte Ausführungsform des erfindungsgemäßen Verfahrens zusätzliche Verfahrensschritte und Unterschritte. Denkbar ist auch ein zu Fig. 3 alternativer Verfahrensablauf, welcher nur einen Teil der in Fig. 3 dargestellten zusätzlichen Verfahrensschritte und/oder Unterschritte aufweist. Selbstverständlich kann auch ein zu Fig. 3 alternativer Verfahrensablauf zusätzliche Verfahrensschritte und/oder Unterschritte aufweisen.

In einem weiteren Verfahrensschritt 44 wird gemäß Fig. 3 zunächst festgelegt, welcher Organstruktur-Typ in den medizinischen Bilddaten segmentiert werden soll. Anschließend umfasst der erste Verfahrensschritt 40 in einem ersten Teilschritt 40-1 eine Prüfung, ob eine genetische Variante, welche zu der morphologischen Variation der Organstruktur führt, in den genetischen Daten des Untersuchungsobjekts vorliegt. Die Prüfung erfolgt gemäß dem festgelegten Organstruktur-Typ. Anschließend kann ein Ergebnis der Prüfung als Eingangsparameter in den Segmentierungsalgorithmus eingehen und der Segmentierungsalgorithmus kann bei der Segmentierung der Organstruktur im weiteren Verfahrensschritt 42 das Ergebnis der Prüfung berücksichtigen.

In einem zweiten Teilschritt 40-2 des ersten Verfahrensschritts 40 umfasst das Erfassen der genetischen Daten ein Erfassen einer Information, in welchem Maße die Organstruktur von der morphologischen Variation in ihrer Morphologie verändert ist. Diese Information kann wiederum als Eingangsparameter in den Segmentierungsalgorithmus eingeht und der Segmentierungsalgorithmus kann bei der Segmentierung der Organstruktur im weiteren Verfahrensschritt 42 die Information berücksichtigen. Selbstverständlich können der erste Teilschritt 40-1 und der zweite Teilschritt 40-2 des ersten Verfahrensschritts 40 auch separat voneinander eingesetzt werden.

In einem weiteren Verfahrensschritt 45 wird ein weiteres patientenspezifisches Merkmal des Untersuchungsobjekts erfasst, wobei das weitere patientenspezifische Merkmal zusätzlich zu den medizinischen Bilddaten und den genetischen Daten in den Segmentierungsalgorithmus eingeht und zumindest ein Merkmal aus der folgenden Liste umfasst:
- ein Alter des Untersuchungsobjekts,
- ein Geschlecht des Untersuchungsobjekts,
- eine Größe des Untersuchungsobjekts,
- ein Gewicht des Untersuchungsobjekts.

Die in Fig. 2-3 dargestellten Verfahrensschritte des erfindungsgemäßen Verfahrens werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das erfindungsgemäße Verfahren auszuführen, wenn das Computerprogramm und/oder die Software in der Recheneinheit mittels einer Prozessoreinheit der Recheneinheit ausgeführt wird.

Verschiedene Möglichkeiten, wie die genetischen Daten besonders geeignet bei der Segmentierung der Organstruktur berücksichtigt werden können, sind in den Ausführungsbeispielen der Fig. 4 bis 7 beschrieben. Die in Fig. 4 bis 7 gezeigten Ausführungsformen des erfindungsgemäßen Verfahrens umfassen im Wesentlichen die Verfahrensschritte 40, 41, 42, 43 der ersten Ausführungsform des erfindungsgemäßen Verfahrens gemäß Fig. 2. Es sei darauf hingewiesen, dass die Fig. 4 bis 7 lediglich spezifische Beispiele zur Illustration des erfindungsgemäßen Vorgehens aufzeigen. Es sind selbstverständlich weitere Anwendungen des erfindungsgemäßen Verfahrens, beispielsweise auf Grundlage von anderen genetischen Variationen oder zur Segmentierung von anderen Organstrukturen, denkbar.

**Fig. 4** zeigt eine erste mögliche Anwendung des erfindungsgemäßen Verfahrens.

Gerade im Bereich der Vermessung von Gehirnstrukturen (Brain Morphometry) zur Diagnose bzw. Früherkennung von degenerativen Gehirnkrankheiten hat die Segmentierung der Gehirnstrukturen einen hohen Stellenwert. Derart soll im in Fig. 4 gezeigten Fall eine Gehirnstruktur segmentiert werden. Der weitere Verfahrensschritt 41 umfasst dazu einen Teilschritt 41-1, in welchem medizinische Bilddaten des Gehirns des Untersuchungsobjekts, insbesondere mittels eines Magnetresonanzgeräts akquirierte Magnetresonanz-Bilddaten, erfasst werden. Weiterhin soll der Segmentierungsalgorithmus eine atlas-basierte Segmentierung unter Verwendung eines Atlas, welcher zumindest eine Atlas-Organstruktur aufweist, einsetzen.

Der erste Verfahrensschritt 40 umfasst im in Fig. 4 gezeigten Fall einen Teilschritt 40-3, in welchem genetische Daten, welche spezifisch für eine morphologische Variation einer Gehirnstruktur sind, erfasst werden. Aus mehreren Schriften sind exemplarisch enge Zusammenhänge zwischen der Morphologie einer Gehirnstruktur und bestimmten genetischen Daten bekannt:
- Stein et al. beschreiben, dass das Vorliegen der intergenetischen Variante rs7294919 zu einer Erhöhung eines Volumens des Hippocampus um 10 Prozent größer als der Populationsmedian führt (Stein et al., Identification of common variants associated with human hippocampal and intracranial volumes, Nat Genet., 2012, 44(5): 552-561).
- Zusammenhänge zwischen subkortikalen Gehirnstrukturen (Hippocampus, Putamen, Nucleus Caudatus, Intrakraniales Volumen) und verschiedenen Genvarianten wurden kürzlich in der Schrift von Hibar et al. publiziert (Hibar et al., Common genetic variants influence human subcortical brain structures, Nature, 2015, 520 (7546) : 224-229).
- Ein genereller Einfluss von Geneigenschaften auf die Größe von verschiedenen Gehirnarealen wurde in der Studie von Thompson et al. nachgewiesen (Thompson et al., Genetic influences on brain structure, Nat Neurosci, 2001, 4(12): 1253-1258).

Derart umfasst der Teilschritt 40-3 eine Prüfung, ob eine genetische Variante, welche zu einer morphologischen Variation der Gehirnstruktur des Untersuchungsobjekt führt, in den genetischen Daten vorliegt. Wenn dies nicht der Fall ist, so kann im weiteren Verfahrensschritt 42 die atlas-basierte Segmentierung der Gehirnstruktur wie üblich durchgeführt werden. Ansonsten kann die atlas-basierte Segmentierung geeignet auf die morphologische Variation der Gehirnstruktur abgestimmt werden.

Hierzu umfasst der weitere Verfahrensschritt 42 einen Teilschritt 42-1, in welchem die Atlas-Organstruktur gemäß dem Vorliegen der durch die genetischen Daten charakterisierten morphologischen Variation deformiert wird. Die Atlas-Organstruktur wird in ihrer Morphologie, beispielsweise ihrer Größe und/oder ihrem Volumen, insbesondere in Vergleich zu weiteren im Atlas enthaltenen Strukturen angepasst. Wenn beispielsweise der Hippocampus segmentiert werden soll und die von Stein et al. beschriebene intergenetische Variante rs7294919 beim Untersuchungsobjekt festgestellt worden ist, so kann die Atlas-Hippocampus-Struktur vor dem Einsatz in der Segmentierung um 10 Prozent vergrößert werden. Der derart angepasste Atlas kann nun besonders geeignet die Vergrößerung des Hippocampus gegenüber dem Populationsmedian berücksichtigen und somit zu besonders exakten Ergebnissen bei der Segmentierung des Hippocampus führen. Beispielsweise kann durch die Anpassung der Größe des Hippocampus an die reale Größe des Hippocampus in den medizinischen Bilddaten eine Registrierung des Atlas auf die medizinischen Bilddaten vereinfacht werden.

Alternativ ist auch das Vorgehen denkbar, dass im Teilschritt 42-1 der für die Segmentierung verwendete Atlas gemäß dem Vorliegen der durch die genetischen Daten charakterisierten morphologischen Variation aus einer Menge an Atlanten ausgewählt wird. Anschließend ist immer noch eine Deformation der Atlas-Organstruktur möglich.

Der weitere Verfahrensschritt 43 umfasst einen Teilschritt 43-1, in welchem die segmentierte Gehirnstruktur bereitgestellt wird. Beispielsweise ist eine Anzeige des Gehirns des Untersuchungsobjekts mit einer farbkodierten Darstellung der segmentierten Gehirnstruktur denkbar. Alternativ oder zusätzlich ist eine Vermessung der segmentierten Gehirnstruktur denkbar, wobei die Ergebnisse der Vermessung in einem Report ausgegeben werden können.

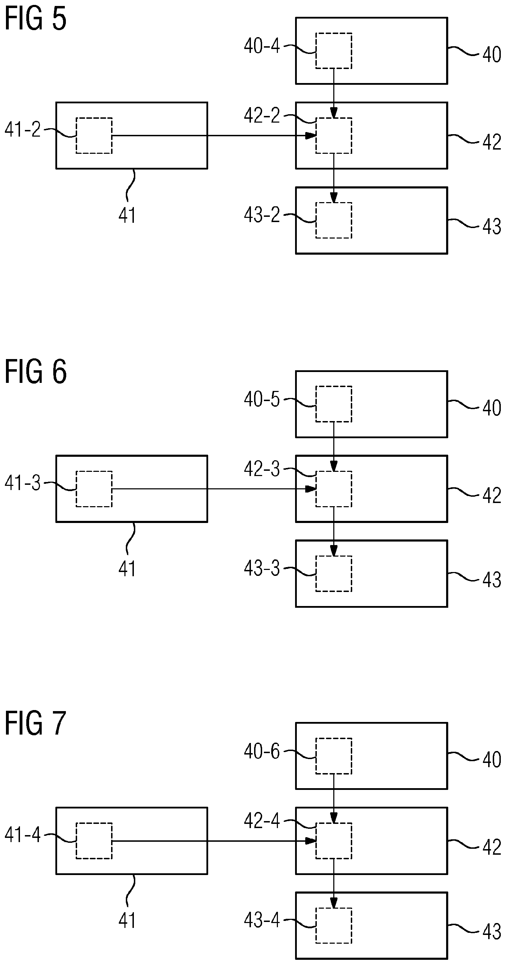

**Fig. 5** zeigt eine zweite mögliche Anwendung des erfindungsgemäßen Verfahrens.

Im in Fig. 5 gezeigten Fall soll die Prostata des Untersuchungsobjekts segmentiert werden. Der weitere Verfahrensschritt 41 umfasst dazu einen Teilschritt 41-2, in welchem medizinische Bilddaten, insbesondere Magnetresonanz-Bilddaten, der Prostata des Untersuchungsobjekts erfasst werden. Weiterhin soll der Segmentierungsalgorithmus ein für die Segmentierung der Organstruktur trainiertes künstliches neuronales Netz einsetzen.

Descazeaud et al. zeigen, dass Genexpressionssignaturen eines Patienten eng mit dem Volumen der Prostata zusammenhängen. Beispielsweise wurde herausgefunden, dass das Gen TEMFF2 beim Vorliegen von einer größeren Prostata auch höher reguliert ist (Descazeaud et al., BPH gene expression profile associated to prostate gland volume, Diagn Mol Pathol, 2008, 17(4): 207-213).

Der erste Verfahrensschritt 40 umfasst im in Fig. 5 gezeigten Fall derart einen Teilschritt 40-4, in welchem genetische Daten, welche spezifisch für eine morphologische Variation der Prostata sind, erfasst werden. Derart wird im Teilschritt 40-4 der Fig. 5 die Regulation des Gens TEMFF2 untersucht. Wenn eine übliche Regulation dieses Gens erkannt wird, so kann im weiteren Verfahrensschritt 42 die Segmentierung wie üblich durchgeführt werden. Bei einer erhöhten Regulation dieses Gens kann die Segmentierung der Prostata geeignet auf die erhöhte zu erwartende Größe der Prostata abgestimmt werden.

Hierzu umfasst der weitere Verfahrensschritt 42 einen Teilschritt 42-2, das für die Segmentierung verwendete künstliche neuronale Netz gemäß dem Vorliegen der durch die genetischen Daten charakterisierten morphologischen Variation verändert wird. Ein Maß für die Hochregulation des Gens TEMFF2 kann direkt als Kennzeichen für eine zu erwartende Größe der Prostata in die Segmentierung mittels des künstlichen neuronalen Netzes eingehen. Das künstliche neuronale Netz kann bei dem Vorliegen einer erhöhten Regulation dieses Gens derart angepasst werden, dass es besonders geeignet zur Segmentierung von besonders großen Prostatadrüsen ist.

Es ist auch denkbar, dass das für die Segmentierung verwendete künstliche neuronale Netz gemäß dem Vorliegen der durch die genetischen Daten charakterisierten morphologischen Variation ausgewählt wird. In diesem Fall stehen für die Segmentierung der Organstruktur ein erstes künstliches neuronales Netz und ein zweites künstliches neuronales Netz bereit, wobei das erste künstliche neuronale Netz mittels eines ersten Trainingskollektivs trainiert worden ist, welches die genetische Variante aufweist, und das zweite künstliche neuronale Netz mittels eines zweiten Trainingskollektivs trainiert worden ist, welches die genetische Variante nicht aufweist, wobei gemäß dem Ergebnis der Prüfung das erste künstliche neuronale Netz oder das zweite künstliche neuronale Netz für die Segmentierung der Organstruktur eingesetzt wird.

Im vorliegenden Fall kann das erste Trainingskollektiv eine höhere Regulierung des Gens TEMFF2 als das erste Trainingskollektiv aufweisen. Derart ist das erste künstliche neuronale Netz besonders zur Segmentierung von größeren Prostatadrüsen als das zweite künstliche neuronale Netz geeignet.

Schließlich ist auch der Anwendungsfall denkbar, dass zunächst eine grobe Segmentierung der Prostata, beispielsweise mittels des künstlichen neuronalen Netzes, durchgeführt wird. Die grobe Segmentierung kann dann mittels geeigneten Randbedingungen verfeinert werden. Der Einfluss der genetischen Daten auf die zu erwartende Größe der Prostata kann bei der groben und/oder bei der feinen Segmentierung als zusätzliche Eingangsinformation verwendet werden bzw. eine zusätzliche Randbedingung bei der Verfeinerung der Segmentierung darstellen.

Der weitere Verfahrensschritt 43 umfasst einen Teilschritt 43-2, in welchem die segmentierte Prostata bereitgestellt wird. Es kann auch ein Volumen der segmentierten Prostata als Maß für ein Vorliegen einer Prostatahyperplasie berechnet werden.

**Fig. 6** zeigt eine dritte mögliche Anwendung des erfindungsgemäßen Verfahrens.

In manchen Anwendungsfällen (z.B. bei der bildbasierten Patientenkontrolle bzw. als Grundlage für folgende Nachverarbeitungen der medizinischen Bilddaten) kann es sinnvoll sein, dass gesamte Körpervolumen des Untersuchungsobjekts zu segmentieren. Derart soll im in Fig. 6 gezeigten Fall das gesamte Körpervolumen des Untersuchungsobjekts segmentiert werden. Der weitere Verfahrensschritt 41 umfasst dazu einen Teilschritt 41-3, in welchem medizinische Bilddaten des gesamten Körpers bzw. eines axialen Körperabschnitts des Untersuchungsobjekts erfasst werden. Der Segmentierungsalgorithmus soll auf eine modell-basierte Segmentierung unter Einsatz eines Körpermodells verwenden.

Heid et al. zeigen, dass die Waist-Hip-Ratio (das Verhältnis zwischen Taillen- und Hüftumfang) eng mit verschiedenen Genvarianten zusammenhängt (Heid et al., Meta-analysis identifies 13 new loci associated with waist-hip ratio and reveals sexual dimorphism in the genetic basis of fat distribution, Nat Genet, 2010, 42(11): 949-960). Der erste Verfahrensschritt 40 umfasst im in Fig. 6 gezeigten Fall einen Teilschritt 40-5, in welchem genetische Daten, welche spezifisch für eine morphologische Variation der Waist-Hip-Ratio sind, erfasst werden.

Dementsprechend kann die mit der Waist-Hip-Ratio verknüpfte Genvariante zusätzlich zur Größe und zum Gewicht des Patienten einen sinnvollen Eingangsparameter für die Segmentierung des gesamten Körpervolumens im Teilschritt 42-3 des weiteren Verfahrensschritts 42 darstellen. Beispielsweise kann gemäß der durch die Genvariante vorhergesagte Waist-Hip-Ratio ein geeignetes Modell für die Körpersegmentierung ausgewählt werden und/oder ein bereits existierendes Modell geeignet angepasst werden. Wenn die Prüfung ergibt, dass das Untersuchungsobjekt eine normale Waist-Hip-Ratio aufweist, da es nicht von der Genvariante betroffen ist, so kann die Körpersegmentierung wie üblich durchgeführt werden.

Der weitere Verfahrensschritt 43 umfasst einen Teilschritt 43-3, in welchem das segmentierte Körpervolumen des Untersuchungsobjekts, insbesondere für eine Weiterverarbeitung, bereitgestellt wird.

**Fig. 7** zeigt eine vierte mögliche Anwendung des erfindungsgemäßen Verfahrens.

Bei der Segmentierung des Herzens oder einer Herzstruktur oder bei der Erkennung von Landmarken im Herzen (z.B. für eine folgende automatische Messplanung) kann die generelle Größe des Herzens einen sinnvollen Eingangsparameter bzw. eine Randbedingung für die Segmentierung darstellen. Derart soll im in Fig. 7 gezeigten Fall eine Herzstruktur, nämlich der linke Ventrikel des Untersuchungsobjekts, segmentiert werden. Der weitere Verfahrensschritt 41 umfasst dazu einen Teilschritt 41-4, in welchem medizinische Bilddaten des Herzens des Untersuchungsobjekts erfasst werden. Weiterhin soll der Segmentierungsalgorithmus ein Region-Growing Verfahren unter Verwendung einer Randbedingung für die Segmentierung des linken Ventrikels einsetzen. Alternativ wäre auch der Einsatz eines Random-Walker Verfahrens denkbar.

Ist das Herz besonders dilatiert (z.B. bei der dilatierten Kardiomyopathie) können konventionelle Segmentierungsalgorithmen nicht mehr funktionieren. Geninformationen des Patienten können auf eine solche Dilatation hinweisen, wie z.B. in der Schrift von Lakdawala et al. beschrieben (Lakdawala et al, Genetic Testing for Dilated Cardiomyopathy in Clinical Practice, J Card Fail, 2012, 18(4): 296-303). Der erste Verfahrensschritt 40 umfasst im in Fig. 7 gezeigten Fall derart einen Teilschritt 40-6, in welchem genetische Daten, welche spezifisch für eine Dilatation des Herzens sind, erfasst werden. Derart umfasst der Teilschritt 40-6 eine Prüfung, ob eine genetische Variante, welche zu einer Dilatation des Herzens führt, in den genetischen Daten vorliegt. Wenn dies nicht der Fall ist, so kann im weiteren Verfahrensschritt 42 die Segmentierung wie üblich durchgeführt werden. Ansonsten kann die Segmentierung der Herzstruktur geeignet auf die Dilatation des Herzens abgestimmt werden.

Hierzu umfasst der weitere Verfahrensschritt 42 einen Teilschritt 42-4, in welchem die Segmentierung des linken Ventrikels geeignet auf die Dilatation der Herzens abgestimmt wird. Die Randbedingung für das Region-Growing Verfahren (oder alternativ für das Random-Walker Verfahren) wird hierbei gemäß der durch die genetischen Daten charakterisierten Dilatation des Herzens festgelegt. Derart kann bei einer zu erwartenden starken Dilatation des Herzens die Randbedingung, welche eine maximale Größe des linken Ventrikels beschreibt, höher gewählt werden.

Der weitere Verfahrensschritt 43 umfasst einen Teilschritt 43-4, in welchem die segmentierte Herzstruktur bereitgestellt wird. Beispielsweise wird der linke Ventrikel hervorgehoben in einer Darstellung des Herzens gezeigt.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen, der durch die Ansprüche definiert wird.

## Patentansprüche

1. Verfahren zum Segmentieren einer Organstruktur eines Untersuchungsobjekts (15) in medizinischen Bilddaten, umfassend folgende Verfahrensschritte:
- Erfassen von genetischen Daten des Untersuchungsobjekts (15), welche eine morphologische Variation der Organstruktur charakterisieren (40),
- Erfassen von medizinischen Bilddaten vom Untersuchungsobjekt (41),
- Segmentieren der Organstruktur in den medizinischen Bilddaten mittels eines Segmentierungsalgorithmus, wobei die genetischen Daten zusätzlich zu den medizinischen Bilddaten als Eingangsparameter in den Segmentierungsalgorithmus eingehen und wobei der Segmentierungsalgorithmus bei der Segmentierung der Organstruktur die morphologische Variation der Organstruktur berücksichtigt (42),
- Bereitstellen der segmentierten Organstruktur (43),
wobei das Erfassen der genetischen Daten eine Prüfung umfasst, ob eine genetische Variante, welche zu der morphologischen Variation der Organstruktur führt, in den genetischen Daten des Untersuchungsobjekts (15) vorliegt (40-1),
wobei für die Segmentierung der Organstruktur ein erstes künstliches neuronales Netz und ein zweites künstliches neuronales Netz bereitstehen, wobei das erste künstliche neuronale Netz mittels eines ersten Trainingskollektivs trainiert worden ist, welches die genetische Variante aufweist, und das zweite künstliche neuronale Netz mittels eines zweiten Trainingskollektivs trainiert worden ist, welches die genetische Variante nicht aufweist, wobei gemäß dem Ergebnis der Prüfung das erste künstliche neuronale Netz oder das zweite künstliche neuronale Netz für die Segmentierung der Organstruktur eingesetzt wird.

2. Verfahren nach Anspruch 1, wobei die morphologische Variation zumindest eines der folgenden morphologischen Merkmale der Organstruktur betrifft:
- eine Größe der Organstruktur,
- eine Form der Organstruktur,
- ein Volumen der Organstruktur,
- eine Lokalisation der Organstruktur im Körper des Untersuchungsobjekts (15).

3. Verfahren nach einem der vorhergehenden Ansprüche, wobei in einem weiteren Verfahrensschritt zunächst festgelegt wird, welcher Organstruktur-Typ in den medizinischen Bilddaten segmentiert werden soll, und wobei die Prüfung gemäß dem festgelegten Organstruktur-Typ erfolgt (44).

4. Verfahren nach einem der vorhergehenden Ansprüche, wobei das Erfassen der genetischen Daten zusätzlich ein Erfassen einer Information umfasst, in welchem Maße die Organstruktur von der morphologischen Variation in ihrer Morphologie verändert ist (40-2), wobei die Information als Eingangsparameter in den Segmentierungsalgorithmus eingeht und der Segmentierungsalgorithmus bei der Segmentierung der Organstruktur die Information berücksichtigt.

5. Verfahren nach einem der vorhergehenden Ansprüche, wobei ein weiteres patientenspezifisches Merkmal des Untersuchungsobjekts (15) erfasst wird, wobei das weitere patientenspezifische Merkmal zusätzlich zu den medizinischen Bilddaten und den genetischen Daten in den Segmentierungsalgorithmus eingeht und zumindest ein Merkmal aus der folgenden Liste umfasst:
- ein Alter des Untersuchungsobjekts (15),
- ein Geschlecht des Untersuchungsobjekts (15),
- eine Größe des Untersuchungsobjekts (15),
- ein Gewicht des Untersuchungsobjekts (45).

6. Verfahren nach einem der vorhergehenden Ansprüche, wobei die zu segmentierende Organstruktur eine der folgenden Organstrukturen ist:
- eine Gehirnstruktur des Untersuchungsobjekts (15),
- eine Prostata des Untersuchungsobjekts (15),
- eine Herzstruktur des Untersuchungsobjekts (15).

7. Recheneinheit (27), umfassend zumindest ein Rechenmodul (33, 34, 35, 36), wobei die Recheneinheit (27) zum Ausführen eines Verfahrens nach einem der vorhergehenden Ansprüche ausgebildet ist.

8. Medizinisches Bildgebungsgerät (11) umfassend eine Recheneinheit (27) nach Anspruch 7.

9. Computerprogrammprodukt, welches direkt in einen Speicher einer programmierbaren Recheneinheit ladbar ist, mit Programmcode-Mitteln, um ein Verfahren nach einem der Ansprüche 1-6 auszuführen, wenn das Computerprogrammprodukt in der Recheneinheit ausgeführt wird.

## Claims

1. Method for segmentation of an organ structure of an examination object (15) in medical image data, comprising the following method steps:
- Acquisition of genetic data of the examination object (15), which characterises a morphological variation of the organ structure (40),
- Acquisition of medical image data from the examination object (41),
- Segmentation of the organ structure in the medical image data by means of a segmentation algorithm, wherein the genetic data is entered into the segmentation algorithm in addition to the medical image data as input parameters and wherein the segmentation algorithm takes account of the morphological variation of the organ structure during the segmentation of the organ structure (42),
- Provision of the segmented organ structure (43),
wherein the acquisition of the genetic data comprises a check as to whether a genetic variant, which leads to the morphological variation of the organ structure, is present in the genetic data of the examination object (15) (40-1), wherein a first artificial neural network and a second artificial neural network are available for the segmentation of the organ structure, wherein the first artificial neural network has been trained by means of a first training collective, which has the genetic variant, and the second artificial neural network has been trained by means of a second training collective, which does not have the genetic variant, wherein in accordance with the result of the check, the first artificial neural network or the second artificial neural network will be used for the segmentation of the organ structure.

2. Method according to claim 1, wherein the morphological variation relates to at least one of the following morphological features of the organ structure:
- A size of the organ structure,
- A shape of the organ structure,
- A volume of the organ structure,
- A localisation of the organ structure in the body of the examination object (15).

3. Method according to one of the preceding claims, wherein it is first determined in a further method step which organ structure type is to be segmented in the medical image data, and wherein the check is made in accordance with the organ structure type determined (44).

4. Method according to one of the preceding claims, wherein the acquisition of the genetic data additionally comprises an acquisition of information as to the extent to which the organ structure is changed by the morphological variation in its morphology (40-2), wherein the information is entered into the segmentation algorithm as input parameters and the segmentation algorithm takes account of the information during the segmentation of the organ structure.

5. Method according to one of the preceding claims, wherein a further patient-specific feature of the examination object (15) is acquired, wherein the further patient-specific feature is entered into the segmentation algorithm in addition to the medical image data and the genetic data and comprises at least one feature from the following list:
- An age of the examination object (15),
- A gender of the examination object (15),
- A size of the examination object (15),
- A weight of the examination object (45).

6. Method according to one of the preceding claims, wherein the organ structure to be segmented is one of the following organ structures:
- A brain structure of the examination object (15),
- A prostate of the examination object (15),
- A heart structure of the examination object (15).

7. Processing unit (27), comprising at least one processing module (33, 34, 35, 36), wherein the processing unit (27) is embodied for carrying out a method according to one of the preceding claims.

8. Medical imaging device (11) comprising a processing unit according to claim 7.

9. Computer program product, which is able to be loaded directly into a memory of a programmable processing unit, with program code means for carrying out the method according to one of claims 1-6, when the computer program product is executed in the processing unit.

## Revendications

1. Procédé de segmentation d'une structure d'organe d'un objet (15) à examiner en des données d'images médicales, comprenant les stades de procédé suivants :
- détection de données génétiques de l'objet (15) à examiner, qui caractérisent (40) une variation morphologique de la structure d'organe,
- détection de données d'images médicales de l'objet (41) à examiner,
- segmentation de la structure d'organe en les données d'images médicales au moyen d'un algorithme de segmentation, les données génétiques étant entrées supplémentairement aux données d'images médicales comme paramètres d'entrée dans l'algorithme de segmentation et l'algorithme de segmentation tenant compte (42), lors de la segmentation de la structure d'organe, de la variation morphologique de la structure d'organe,
- mise à disposition de la structure (43) d'organe segmentée, la détection des données génétiques comprenant un contrôle sur le point de savoir, si une variante génétique, qui donne la variation morphologique de la structure d'organe, est présente (40-1) dans les données génétiques de l'objet à examiner,
dans lequel, pour la segmentation de la structure d'organe, on dispose d'un premier réseau neuronal artificiel et d'un deuxième réseau neuronal artificiel, le premier réseau neuronal artificiel ayant subi un apprentissage au moyen d'un premier collectif d'apprentissage, qui a les variantes génétiques, et le deuxième réseau neuronal artificiel ayant été soumis à un apprentissage au moyen d'un deuxième collectif d'apprentissage, qui n'a pas les variantes génétiques, dans lequel, suivant le résultat du contrôle, on utilise le premier réseau neuronal artificiel ou le deuxième réseau neuronal artificiel pour la segmentation de la structure d'organe.

2. Procédé suivant la revendication 1, dans lequel la variation morphologique concerne au moins l'une des caractéristiques morphologiques suivantes de la structure d'organe :
- une dimension de la structure d'organe,
- une forme de la structure d'organe,
- un volume de la structure d'organe,
- une localisation de la structure d'organe dans le corps de l'objet (15) à examiner.

3. Procédé suivant l'une des revendications précédentes, dans lequel, dans un autre stade du procédé, on fixe d'abord le type de structure d'organe, qui doit être segmenté en les données d'images médicales, et dans lequel le contrôle s'effectue en fonction du type de structure d'organe fixé.

4. Procédé suivant l'une des revendications précédentes, dans lequel la détection des données génétiques comprend, en outre, une détection d'une information de la mesure dans laquelle la structure d'organe est modifiée (40-2) dans sa morphologie par la variation morphologique, l'information étant entrée comme paramètre d'entrée dans l'algorithme de segmentation et l'algorithme de segmentation tenant compte de l'information lors de la segmentation de la structure d'organe.

5. Procédé suivant l'une des revendications précédentes, dans lequel on détecte une autre caractéristique spécifique au patient de l'objet (15) à examiner, l'autre caractéristique spécifique au patient étant, en plus des données d'images médicales et des données génétiques, entrée dans l'algorithme de segmentation et comprenant au moins une caractéristique dans la liste suivante :
- un âge de l'objet (15) à examiner,
- un genre de l'objet (15) à examiner,
- une dimension de l'objet (15) à examiner,
- un poids de l'objet (45) à examiner.

6. Procédé suivant l'une des revendications précédentes, dans lequel la structure d'organe à segmenter est l'une des structures d'organe suivantes :
- une structure de cerveau de l'objet (15) à examiner,
- une prostate de l'objet (15) à examiner,
- une structure de coeur de l'objet (15) à examiner.

7. Unité (27) d'ordinateur, comprenant au moins un module (33, 34, 35, 36) d'ordinateur, l'unité (27) d'ordinateur étant constituée pour exécuter un procédé suivant l'une des revendications précédentes.

8. Appareil (11) d'imagerie médicale, comprenant une unité (27) d'ordinateur suivant la revendication 7.

9. Produit de programme d'ordinateur, qui peut être chargé directement dans une mémoire d'une unité d'ordinateur programmable, comprenant des moyens de code de programme, pour exécuter un procédé suivant l'une des revendications 1 à 6, lorsque le produit de programme d'ordinateur est réalisé dans l'unité d'ordinateur.
